# EUROPEAN PATENT SPECIFICATION

(11) **EP 1 857 441 B1**
(45) Date of publication and mention of the grant of the patent: **14.10.2009**
(21) Application number: 07009776.1
(22) Date of filing: 16.05.2007
(51) Int. Cl.: C07C 403/24, C09B 61/00

(54) **Method of separating and purifying xanthophyll fatty acid esters from marigold oleoresin**
Verfahren zur Trennung und Reinigung von xanthophyll-Fettsäurestern aus dem Fettharz von Ringelblumen
Procédé de séparation et de purification d'esters d'acide gras de xanthophyll provenant d'oléorésine de soucis

(30) Priority: 17.05.2006 CN 200610026613
(43) Date of publication of application: 21.11.2007
(73) Proprietor: ZheJiang Medicine Co., Ltd. Xinchang Pharmaceutical Factory, Xinchang Zhejiang 312500 (CN)
(72) Inventor: Xu, Xinde, Zhejiang, 312500 (CN); Chen, Boqiu, Zhejiang, 312500 (CN); Zhou, Di, Zhejiang, 312500 (CN); Ye, Shuangming, Zhejiang, 312500 (CN); Huang, Shengcong, Zhejiang, 312500 (CN); Shao, Bin, Zhejiang, 312500 (CN)
(74) Representative: Meyer-Dulheuer, Karl-Hermann

(56) References cited:
- EP-A2- 1 325 943
- WO-A-02/094772
- WO-A1-99/20587
- US-A- 4 048 203
- US-A1- 2003 229 142

## Description

This invention belongs to the domain of phytochemistry, involving the method of extracting and purifying **lutein** fatty acid ester from marigold oleoresin.

Description of the Related Art

Carotenoid is a yellow to red substance existing widely in the nature. Various kinds of carotenoids are found in various fruits and vegetables. For example, the contents of β-carotene in carrot, **lutein** in marigold, zeaxanthin in strawberry, lycopene in tomato, and capsarubin and capsicum pigment in capsicum vegetable are all high. In addition, the carotenoid and xanthophyll contents are also high in certain fuscous fruit and vegetable, yolk, carapace of fish, birds, seaweed, and bacteria.

In recent years, experiments on animals and humans proved the benefits of carotenoids. In general, carotenoids can be divided into two sub-classes: (1) **xanthophylis** or oxygenous carotenoids with strong polarity, such as **lutein,** zeaxanthin and astaxanthin, and **(2)** non-polar hydrocarbon carotenoids, such as β-carotene, and lycopene. These two kinds of carotenoids contain at least nine conjugated double bonds which result in the coloring characteristic of carotenoids and their strong anti-oxidation effects exploited for preventing and curing diseases, such as cancer, arteriosclerosis, cataracts, age-related macular degeneration, and so on. Moreover, carotenoids can eliminate oxygen free radicals, prevent free radicals from generating, and limit the damage effect of free radical oxidation.

Among carotenoids, **lutein** and zeaxanthin attract special attention because of their potential capability for preventing age-related macular degeneration (AMD).

**Lutein** and zeaxanthin are the only carotenoids existing in the spot region of the human's retina, which are closely related to the visual acuity (Bone et al.Invest Ophthamal. Vs.Sci. 34:2033-2040, 1993). Eating fruits and vegetables which are rich in **lutein** and zeaxanthin can reduce up to 43% the risk of suffering from age-related macular degeneration (Seddon et al.J.Am.Med.Assoc.272:1413-1420, 1994). In addition, the metabolic pathway of these compounds in preventing age-related macular degeneration is known. FDA considers **lutein** and zeaxanthin as GARS. Therefore, these carotenoid can be used alone (or mixed with other substances) as nutritional supplements and food colorants, and be applied in the clinical prevention of age-related macular degeneration, cancer, and so on.

Many fruits and vegetables (such as spinach, cauliflower, cabbage, com and so on) are rich in **lutein.** Among them, calendulas and marigolds are the richest. Of course, there are also other carotenoids existing in these vegetables. Lutein in vegetable is generally esterified to the mono-ester and the di-ester with the C12-C18 long chain fatty acid, such as myristic acid, oleic acid, linoleic acid and palmitic acid. These natural **xanthophyll** esters have the characteristics of stability and bioavailblility. They can be used as diet supplements, food additives and colorants. **Lutein** esters are more liposoluble than the free **lutein** crystal, which will contribute to their broad application.

At present, there are many methods and patents for extracting **xanthophyll** ester from marigold. One method described in US Pat. No. 4,048,203 involves dissolving marigold oleoresin in a hot alkyl-alcohol having not more than 4 C-atoms, then cooling down the solution to deposit the **lutein** fatty acid ester, separating the deposition by filtration, and then desiccating in vacuum. The content of the **lutein** fatty acid ester in the residue obtained by this method is only 51 %, which is not high.

Another method described in CN Pat. No. 1,120,565 A involves treating a saponified oleoresin from marigolds with petroleum ether. However, the content of the title product by this method is only 12.0%, and the yield is low.

Another method described in US Pat. No. 6,191,293 involves extracting marigold with a hydrocarbon solvent, and adding an alcohol to obtain the extract. However, the purity of the **lutein** fatty acid ester obtained by this method from oleoresin is variable due to a varying quality of the raw material, and thus it is impractical for industrial production.

Another method described in CN1,432,567 A involves extracting **lutein** oleoresin with acetone, and then crystallizing the concentrated acetone mother liquor with the help of butanol or isopropanol to obtain a high-content **lutein** fatty acid ester residue. Using this method the yield is low on one hand; on the other hand, it is difficult to obtain a material that would have an acceptably low level of residual solvent for use in food applications because of the high boiling point of butanol. In addition, the ratio of trans to cis-isomers of the **xanthophyll** fatty aid ester in the product is affected by the raw materials. If the cis-isomer content in the raw material is high, the bioactivity of the product will be reduced.

Another method described in US Pat No. 6,737,535 B2 involves removing impurities by extracting **lutein** oleoresin with ketone-based reagents. The alltrans isomer content in the product is high, but carotenoid content is only 60%, and the yield is only 30%, which is comparatively low. Hence such techniques are not suitable for the large-scale industrial production. It is also unsafe to apply products having only 60% purity in a food additive or health food because they contain too many unknown impurities.

As stated, these methods have disadvantages. For instance, the yield is low and the purity is insufficient. The quality of the products is affected by the quality of the raw material, i.e., the ratio of the trans-isomer of **lutein** to the cis-isomer cannot be adjusted during isolation. The high boiling point of the organic solvent used in these methods brings difficulty in isolation in subsequent processes. The high-content of the residual solvent makes these methods difficult to be applied in industrial production. Accordingly, it is necessary to find a new technique to obtain a high-content **lutein** ester residue.

The technical problems to resolve in this invention are to surmount the disadvantages mentioned above, and to study and design a method of producing high-content **lutein** ester residue, which is characterized by high yield, lower amount of residual organic solvents, stability of techniques and suitability for large-scale industrial production.

This invention introduces a method of extracting **lutein** esters and **lutein** fatty acid diesters from marigold oleoresin.

The method of this invention comprises: blending a marigold oleoresin with a mixture of n-hexane and an alcohol to form a homogeneous solution, allowing the solid particles to dissolve for a certain time, cooling down the mixture, crystallizing, filtering, and dissolving the insoluble filter cake with n-hexane by reflux, cooling down again, crystallizing to obtain the insoluble substances, drying the substance in vacuum to get a product having **lutein** ester content of over 80% by weight. The content of the all-trans **lutein** fatty acid ester is more than 90%, and the **lutein** ester yield is about 70%

The invention comprised technical processes as listed below:
a. Blend the marigold oleoresin with the mixed solvent composed of n-hexane and alcohol, dissolve for a certain time by reflux, cool down and crystallize;
b. Obtain crystals by routine methods, wherein the alcohol soluble impurities in the marigold oleoresin are removed;
c. Dissolve the crystals in a certain amount of n-hexane, cool down and recrystallize. Obtain crystals by routine methods again.
d. Eliminate the residual solvent by desiccating crystals obtained in step c) in vacuum, until the residual solvent reaches a food-acceptable standard.

The embodiments of the invention are described in detail as below:

In the invention, under the temperature of 40°C -85°C, the marigold oleoresin is dissolved into the mixed solvent of ethyl alcohol and n-hexane to form a homogeneous solution. Normally, one weight part of oleoresin is placed into 1.0-15 weight parts of mixed solvent. The percentage of ethyl alcohol in the mixed solvent is 10-70%, and the percentage of n-hexane is 90%-30%. Agitate for a sufficient amount of time (generally for 1.0-5.0 hours), the **lutein** fatty acid ester and other impurities such as wax, resin, other carotenoid and pigment will dissolve and be dispersed into the solvent and form a homogeneous solution.

Cool down the solution to 10-40°C and crystallize. Separate the crystals from the solution by normal methods such as centrifugation, press filtration and suction filtration. Then, the alcohol soluble impurities are transferred to the mother liquid and the **lutein** fatty acid ester is purified. Because the oleoresin remains dissolved in the alcohol solvent for enough time, this helps to increase the content of the all-trans **lutein** fatty acid ester isomer in the products. If the content of all-trans **lutein** ester in the marigold oleoresin is not high enough, a prolonged exposure to a solvent can transform part of the cis-isomer of **lutein** fatty acid ester into the transisomer, which will improve the bioactivity of the **lutein** fatty acid ester. The alcohol used in the reaction can be methanol, ethanol, propanol or isopropanol. The ethyl alcohol is preferred.

By such two steps, the content of **lutein** fatty acid ester in the intermediate substances can and does reach about 70% by weight. The percentage of all-trans isomer is higher than 90%; the content of zeaxanthin fatty acid ester is about 5%; and the remaining parts are the cis-isomer and other carotenoids.

According to the method of the invention, dissolve the intermediate crystal to n-hexane, agitate and reflux at 50-85°C for 1-2 hours to disperse it sufficiently. Normally, one weight part of solid is dissolved in 0.5-10 weight parts of n-hexane. 2-6 weight parts are preferential.

Cool down the mixed solution to 0-20°C slowly. Cooling down to 3-10°C is preferred. In general, the time of cooling is about 2-5 hours. In the process of cooling, selecting from one to several solvents, such as THF, isopropanol, ethanol or their mixtures, and adding these to the solution. Adding while agitating is preferred. These solvents can help to deposit the insoluble solids, which will increase the operating efficiency and improve the final yield of products. The volume of isopropanol added is as much as 0.1-3 volumes of n-hexane; 0.5-2 volumes are preferred. The volume of ethanol added is as much as 0.1-3 volumes of n-hexane; 0.8-2 volumes are preferred. The volume of THF is as much as 0.01-2 volumes of n-hexane; 0.01-1 volumes **are** preferred.

In these steps, the addition of methanol, ethanol, water or their mixtures may be under room temperature, but the same temperature as that of the cold solution is preferential, and there is no strict requirement for the speed of solvent adding.

Keep the cold mixture under the given temperature and agitate the solution slowly for 0.5-2.0 hours; 1.0-1.5 hours is preferred. At last, separate the insoluble solid by normal methods such as centrifuge, press filtration and suction filtration. Recover the mixed solvent in the mother liquor and discard the impurities.

Desiccate the solid in a vacuum oven to remove the remaining solvents. The desiccating temperature is 20-55°C; 40-50°C is preferred. The vacuum pressure is from -0.06 - 0.095 MPa (-0.090 - 0.095MPa is preferred). Desiccate the solid until the residual n-hexane can no longer be detected in the products.

According to the methods of the invention, the final product, **lutein** fatty acid ester condensate is a yellow to pale-brown solid. The content of **lutein** fatty acid ester is not less than 70%, and generally higher than 80%. The analysis by high performance liquid chromatography (HPLC) shows that it contains 90-95% of alltrans **lutein** ester, 0.1-1.0% of its geometric isomer, 2.0-7.0% of all-trans zeaxanthin and less than 1.0% of other carotenoids, which is incidentally also the requirement of the foods and safe for eating. Moreover, the **lutein** fatty acid ester yield reaches up to about 70%, and is much higher than in other existing methods. What is more important, the boiling point of the organic solvents used in the production is lower than 80°C, so it is easy to remove and its low residual amount can meet the safety standard of food industry. The **lutein** fatty acid ester can be used as food colorant because of its bright color, directly or microencapsulated into a dry powder, and added to diet supplements.

The advantages of this invention are listed below: (1) the organic solvents applied in the production is absolute ethyl alcohol and n-hexane, which have low boiling point and can be easily removed during vacuum drying. As a result, the residual solvent amount is low and the product can be safely applied as food additive, diet supplement and colorant; (2) in the final product, the **lutein** ester content is up to 80%, and the other natural impurities are little. The yield of **lutein** is about 70%; (3) In the carotenoid part of the product, there are more than 90% of alltrans **lutein** ester, and 5% of the cis-isomer, and the rest is a small amount of zeaxanthin ester and carotenoid substances. The activity of the product is high; (4) the operating procedure is simple; and the methods are suitable for large-scale industrial production. With these advantages, this invention is superior to the previously disclosed methods. The technique introduced in this invention is economical, and suitable for large-scale industrial production. These advantages and others are described in detail in the later ensuing description.

This invention involves the method of **separating** and purifying **lutein** fatty acid ester from natural resources, such as marigold, broccoli, cabbage, spinach and corn. In these raw materials, the marigold oleoresin has relatively high-content of **lutein** ester, so they are the preferential raw materials.

In this invention, the edible marigold oleoresin extracted by n-hexane is the raw material to separate high-content all-trans **lutein** ester. Because of the difference of types, planting conditions, time of harvesting and extracting method, the oleoresin content of **lutein** fatty acid ester is in a range of 5-30%. In addition, there are a few other carotenoids, such as all-trans zeaxanthin, α-cryptoxanthin, β-cryptoxanthin, β-carotene, etc. In addition, this method is not influenced by the quality of marigold oleoresin, especially the variable content of **lutein** fatty acid ester.

Example 1:

Blended 1000g marigold oleoresin (overall **lutein** fatty ester content is 31.8%) is mixed with 5000 mL absolute ethyl alcohol and 3000 mL of n-hexane. Heated the mixture to 80°C, then agitated the solution and refluxed till it became a uniform solution. Kept the solution for 3 hours under the temperature of 80°C, and then cooled down slowly the solution to 25°C within 2.5 hours. Kept the temperature for crystallization. Separated the crystals by centrifuge filtration. The intermediate was weighted to be 401 g. UV detection results showed that the **lutein** fatty ester content was 71 % by weight. Took a sample for saponification and detected by HPLC method, the results showed that there was 92% of the all-trans lutein and 7% of zeaxanthin, the rest were few cis-isomers and carotenoid substances.

Blended the obtained 400.0 g of intermediate crystals with 1000 mL n-hexane, and heated to 70°C under agitation. After refluxing for 2 hours, cooled down the solution to 10°C for crystallization. During the cooling process, added 80 mL THF slowly. Separated solid insoluble substance by suction filtration and washed by rinsing with 100 mL of absolute ethyl alcohol for three times.

The solid insoluble substance was dried in by vacuum drying oven under a temperature of 50°C and a pressure of-0.095 MPa. Monitoring the residual solvent by head space gas chromatography during drying process till n-hexane could not be detected. 276.0 g products were obtained. UV detection result showed that the **lutein** fatty acid ester content was 82.1 %. Took a sample for saponification and detection by HPLC method. The results showed that there was 92.5% of the all-trans lutein and 7% of zeaxanthin, the rest was few cis-isomers and carotenoid substances. The overall xanthophyll fatty acid ester yield was 71.2%.

The product was free of poisonous organic solvents, and was suitable for application in nutritional supplements and food additives. The application form of this crystal could be oil suspension (mixed and emulsified with vegetable oil), pellet (microcapsule, frozen form spray), or dry powder (microcapsule, desiccated from spray).

Example 2:

Blended 1000g marigold oleoresin (overall **lutein** fatty ester content was 22.7%, among them there was 72.7% of all-trans **lutein)** with a mixed solvent of 5600 mL absolute ethyl alcohol and 2000 mL n-hexane. Heated the mixture to a temperature of 80°C, and then stirred up the mixture and refluxed until it became a uniform solution. Kept the solution for 5 hours under the temperature of 80 °C, and then cooled down the solution to 30 °C within 2 hours. Kept the temperature for crystallization. Separated the crystals by press filtration. The intermediate was weighted to be 392.5 g. UV detection results showed that the **lutein** fatty acid ester content was 68.5% by weight. Took a sample for saponification and detection by HPLC method. The results showed that there was 90.5% of the all-trans lutein and 8.3% of zeaxanthin; the rest was few cis-isomers and carotenoid substances.

Blended the obtained 390.0 g of intermediate crystals with 1500 mL n-hexane, and heated to 70°C under agitation. After reflux for 2 hours, cooled down the solution to 15°C for crystallization. During the cooling process, added 60 mL isopropanol under a temperature of 10°C. Separated solid insoluble substance by suction filtration and washed by rinsing with 100 mL absolute ethyl alcohol for three times.

Desiccated the solid insoluble substance by vacuum drying oven at a temperature of 50°C and a vacuum pressure of-0.095MPa. Monitored the residual solvent amount by head space gas chromatography during the drying process till n-hexane could no longer be detected. 195.0 g dried products were obtained. UV detection result showed that the **lutein** fatty acid ester content was 79.6%. Took a sample for saponification and detection by HPLC method. The results showed that there was 91.5% of the all-trans lutein and 8.1% of zeaxanthin, the rest was few cis-isomers and carotenoid substances. The overall xanthophyll fatty acid ester yield was 38.4%.

## Claims

1. A method to extract and purify xanthophyll fatty acid ester from marigold oleoresin, comprising:
(1) dissolving marigold oleoresin in a mixture of ethyl alcohol and n-hexane at 40°C - 85°C, agitating for 1.0 - 5.0 hours, and obtaining a homogeneous solution;
(2) recrystallizing from the solution in step (1) by agitating slowly under the temperature of 10°C - 40°C, obtaining purified solid crystals;
(3) dissolving the solid crystals from step (2) in n-hexane, agitating and refluxing for 1-2 hours under the temperature of 50°C - 85°C;
(4) cooling down the n-hexane solution obtained in step (3) to 0 - 20°C; during the cooling process, agitating and adding tetrahydrofuran (THF) or an alcohol, such as ethanol or isopropanol, or a mixture of THF and alcohol; keeping the solution for 0.5 - 2.0 hours after a deposition appears; separating to remove the solvent and obtain the deposited solid;
(5) desiccating the solid from step (4) at a temperature of 20-55°C and vacuum pressure of -0.090-0.095MPa; removing the solvent and obtaining a high-content **lutein** fatty acid ester residue.

2. The method of claim 1, wherein the volume ratio of the marigold oleoresin to the mixture of ethyl alcohol and n-hexane in step (1) is 1:1.0∼1.5; the ethyl alcohol percentage in the mixed solvent is 10-70%, the n-hexane percentage is 90~30%; and the ethyl alcohol is selected from methanol, ethanol, propanol or isopropanol.

3. The method of claim 1, wherein the volume ratio of the solid to n-hexane in step (3) is 1:0.5-10.

4. The method claim 1, wherein the volume ratio of the solid mentioned in step (3) to n-hexane is 1:2-6.

5. The method of claim 1, wherein the mixture in step (4) is cooled down slowly to 3-10°C.

6. The method of claim 1, wherein the solvent added to the n-hexane solution in step (4) is THF, ethanol, isopropanol, or mixtures; the volume ratio of n-hexane to the added solvents is: for isopropanol: 0.1-3; for ethanol: 0.1-3; and for THF: 0.01-2.

7. The method of claim 1, wherein the solvent added to the n-hexane solution in step (4) is water or ethanol or methanol or their mixtures; and the volume ratio of the solvent to n-hexane is: for methanol: 0.5-2; for ethanol: 0.8-2; and for water: 0.01-1.

8. The method of claim 1, wherein mixture in step (4) is kept cold under the given temperature and the mixture is agitated slowly for 1.0-1.5 hours.

9. The method claim 1, wherein the solid is further dried in the vacuum oven to remove the remaining solvent in step (5) at the temperature of 40-50°C; and the vacuum pressure is 0.090-00.095MPa.

10. The method of claim 1, wherein the obtained high-content **lutein** fatty acid ester residue can be used as food additive, diet supplement or colorant.

## Patentansprüche

1. Verfahren zum Extrahieren und Reinigen von Xanthophyll-Fettsäureestern aus dem Fettharz von Ringelblumen, beinhaltend folgende Verfahrensschritte:
(1) Auflösen des Fettharzes von Ringelblumen in einer Mischung aus einem Alkohol und n-Hexan bei 40°C - 85°C, Rühren für 1,0 - 5,0 Stunden und Erhalten einer homogenen Lösung;
(2) Rekristallisieren aus der Lösung aus Schritt (1) durch langsames Mischen bei einer Temperatur von 10°C - 40°C, wobei gereinigte feste Kristalle erhalten werden;
(3) Auflösen der festen Kristalle aus Schritt (2) in n-Hexan, Rühren unter Rückfluss für 1,0 - 2,0 Stunden bei einer Temperatur von 50°C - 85°C;
(4) Abkühlen der in Schritt (3) erhaltenen n-Hexan-Lösung auf 0°C - 20°C; Rühren und Zugabe von Tetrahydrofuran (THF) oder eines Alkohols, wie beispielsweise Ethanol oder Isopropanol, oder einer Mischung aus THF und Alkohol während des Abkühlens; Behalten der Lösung für 0,5 - 2,0 Stunden nach Auftreten eines Niederschlags; Abtrennen zur Entfernung des Lösungsmittels und Gewinnung des niedergeschlagenen Feststoffes;
(5) Trocknen des Feststoffes aus Schritt (4) bei einer Temperatur von 20°C - 55°C, Trocknen unter Vakuum; Entfernen des Lösungsmittels und Erhalten eines Rückstandes mit einem hohen Gehalt an Lutein-Fettsäureester.

2. Verfahren nach Anspruch 1, **dadurch gekennzeichnet, dass** das Volumenverhältnis von Ringelblume-Fettharz zur Mischung aus einem Alkohol und n-Hexan in Schritt (1) 1:1,0 - 1,5 beträgt; der Alkoholgehalt in der Lösungsmittel-Mischung 10% - 70% beträgt, der n-Hexan-Gehalt 90% - 30% beträgt; und der Alkohol aus einem der nachfolgenden ausgewählt ist: Methanol, Ethanol, Propanol oder Isopropanol.

3. Verfahren nach Anspruch 1, **dadurch gekennzeichnet, dass** das Volumenverhältnis von Feststoff zu n-Hexan in Schritt (3) 1:0,5 - 10 beträgt.

4. Verfahren nach Anspruch 1, **dadurch gekennzeichnet, dass** das Volumenverhältnis von Feststoff in Schritt (3) zu n-Hexan 1:2 - 6 beträgt.

5. Verfahren nach Anspruch 1, **dadurch gekennzeichnet, dass** die Mischung in Schritt (4) langsam auf 3°C - 10°C abgekühlt wird.

6. Verfahren nach Anspruch 1, **dadurch gekennzeichnet, dass** das zur n-Hexan-Lösung in Schritt (4) zugegebene Lösungsmittel THF, Ethanol, Isopropanol oder eine Mischung ist; das Volumenverhältnis von n-Hexan zum zugegebenen Lösungsmittel für Isopropanol 0,1 - 3; für Ethanol 0,1 - 3; und für THF 0,01 - 2 beträgt.

7. Verfahren nach Anspruch 1, **dadurch gekennzeichnet, dass** das zur n-Hexan-Lösung in Schritt (4) zugegebene Lösungsmittel Wasser, Ethanol, Methanol oder eine Mischung derselben ist; und das Volumenverhältnis von Lösungsmittel zu n-Hexan für Methanol 0,5 - 2; für Ethanol 0,8 - 2; und für Wasser 0,01 - 1 beträgt.

8. Verfahren nach Anspruch 1, **dadurch gekennzeichnet, dass** die Mischung in Schritt (4) bei der festgelegten Temperatur kalt gehalten wird und die Mischung langsam für 1,0 - 1,5 Stunden gerührt wird.

9. Verfahren nach Anspruch 1, **dadurch gekennzeichnet, dass** der Feststoff weiter in einem Vakuum-Ofen bei einer Temperatur von 40°C - 50°C getrocknet wird, um das restliche Lösungsmittel aus Schritt (5) zu entfernen.

10. Verfahren nach Anspruch 1, **dadurch gekennzeichnet, dass** der erhaltene Rückstand mit einem hohen Gehalt an Lutein-Fettsäureester als Nahrungsmittel-Zusatz, diätetischer Zusatz oder Farbstoff eingesetzt werden kann.

## Revendications

1. Procédé d'extraction et de purification d'esters d'acide gras de xanthophylle provenant d'oléorésine de soucis, comprenant les étapes suivantes:
(1) dissoudre l'oléorésine de soucis dans un mélange d'alcool et de n-hexane à une température comprise entre 40 et 85°C, agiter entre 1,0 et 5,0 heures et obtenir une solution homogène;
(2) recristalliser de la solution obtenu à l'étape (1) en remuant lentement à une température comprise entre 10 et 40°C, obtenir des cristaux solides purifiés;
(3) dissoudre les cristaux solides obtenus à l'étape (2) dans du n-hexane, agiter en reflux entre 1,0 et 2,0 heures à une température comprise entre 50 et 85°C;
(4) refroidir la solution de n-hexane obtenue à l'étape (3) jusqu'à une température comprise entre 0 et 20°C; agiter et ajouter du tétrahydrofurane (THF) ou un alcool, comme l'éthanol ou l'isopropanol, ou un mélange de THF et d'alcool pendant le refroidissement; garder la solution entre 0,5 et 2,0 heures après l'apparition d'un sédiment; séparer pour éliminer le solvant et obtenir la matière solide sédimentée;
(5) sécher la matière solide obtenue à l'étape (4) à une température comprise entre 20 et 55°C, sécher sous vide; extraire le solvant et obtenir un résidu à teneur élevée d'ester d'acide gras de lutéine.

2. Procédé selon la revendication 1, **caractérisé en ce que** le rapport volumique entre l'oléorésine de soucis et le mélange d'alcool et de n-hexane de l'étape (1) est compris entre 1:1,0 et 1:1,5; la teneur en alcool dans le mélange de solvant est comprise entre 10 et 70%, la teneur de n-hexane est comprise entre 90 et 30%; et l'alcool est sélectionné parmi les suivants: méthanol, éthanol, propanol ou isopropanol.

3. Procédé selon la revendication 1, **caractérisé en ce que** le rapport volumique entre la matière solide et le n-hexane dans l'étape (3) est compris entre 1:0,5 et 1:10.

4. Procédé selon la revendication 1, **caractérisé en ce que** le rapport volumique entre la matière solide dans l'étape (3) et le n-hexane est compris entre 1:2 et 1:6.

5. Procédé selon la revendication 1, **caractérisé en ce que** le mélange dans l'étape (4) est refroidi lentement jusqu'à une température comprise entre 3 et 10°C.

6. Procédé selon la revendication 1, **caractérisé en ce que** le solvant ajouté à la solution de n-hexane dans l'étape (4) est du THF, de l'éthanol, de l'isopropanol ou un mélange; le rapport volumique entre le n-hexane et le solvant ajouté est compris pour l'isopropanol entre 0,1 et 3; pour l'éthanol entre 0,1 et 3; et pour le THF entre 0,01 et 2.

7. Procédé selon la revendication 1, **caractérisé en ce que** le solvant ajouté à la solution de n-hexane dans l'étape (4) est de l'eau, de l'éthanol, du méthanol ou un mélange de ceux-ci; et le rapport volumique entre le solvant et le n-hexane est compris pour le méthanol entre 0,5 et 2; pour l'éthanol entre 0,8 et 2; et pour l'eau entre 0,01 et 1.

8. Procédé selon la revendication 1, **caractérisé en ce que** le mélange dans l'étape (4) est maintenu à la température indiquée et agité lentement entre 1,0 et 1,5 heures.

9. Procédé selon la revendication 1, **caractérisé en ce que** la matière solide est séchée à nouveau pour éliminer les restes de solvant de l'étape (5) dans un four à vide à une température comprise entre 40 et 50°C.

10. Procédé selon la revendication 1, **caractérisé en ce que** le résidu obtenu à teneur élevée d'ester d'acide gras de lutéine peut être utilisé comme supplément alimentaire, supplément diététique ou comme colorant.
